(19) Europäisches Patentamt
European Patent Office
Office européen des brevets

(11) Publication number: 0 300 741
A2

(12) **EUROPEAN PATENT APPLICATION**

(21) Application number: 88306615.1

(51) Int. Cl.[4] C07K 7/10 , A61K 37/02

(22) Date of filing: 20.07.88

(30) Priority: 20.07.87 US 53286

(43) Date of publication of application:
25.01.89 Bulletin 89/04

(84) Designated Contracting States:
BE CH DE ES FR GB IT LI NL

(71) Applicant: ORTHO PHARMACEUTICAL CORPORATION
U.S. Route 202 P.O. Box 300
Raritan New Jersey 08869-0602(US)

(72) Inventor: Goldstein, Gideon
30 Dorison Drive
Short Hills, NJ 07078(US)
Inventor: Audhya, Tapan
473 Foothill Road
Bridgewater NJ 08807(US)

(74) Representative: Jones, Alan John et al
CARPMAELS & RANSFORD 43 Bloomsbury Square
London, WC1A 2RA(GB)

(54) **Human thymopoietin.**

(57) The present invention relates to human thymopoietin substantially free of non-human thymopoietin native matter, to therapeutic compositions containing it and to the use of such thymopoietin or compositions in modulating the immune system.

EP 0 300 741 A2

# HUMAN THYMOPOIETIN

## Background of the Invention

### 1. Field of the Invention

The invention relates generally to a newly isolated protein and more particularly to a protein either isolated in purified form from the human thymus or chemically synthesized, and to therapeutic compositions and methods for employing the same.

### 2. Description of the Prior Art

Thymopoietin (TP) is a polypeptide hormone of the thymus with pleiotropic actions on prothymocytes, mature T cells, the nicotinic acetylcholine receptor and pituitary corticotrophs. Accordingly, there has been great interest in such substances and a large body of articles and patents have been published relating to such as are present in and isolated from bovine thymus, as well as chemically synthesized peptides mimicking the properties of the naturally occurring substances. In particular, see for example, Goldstein, G. (1974) Nature (London) 247, 11-14; Basch, R.S. and Goldstein, G. (1974) Proc. Natl. Acod. Sci. U.S.A. 71, 1474-1478; Scheid, M.P., Goldstein, G. and Boyse, E.A. (1978) J. Exp. Med. 147, 1727-1743; Scheid, M.P., Goldstein, G. and Boyse, E.A. (1975) Science 190, 1211-1213; Ranjes, G.E. Scheid, M.P., Goldstein, G. andBoyse, E.A. (1982) J. Exp. Med. 156, 1057-1064; Vankatasubramanian, K., Andhya, T. and Goldstein, G. (1986) Pric. Nat. Acul. Sci. U.S.A. 83, 3171-3174; Malaise M.G., Harz-Hazelstein, M.T., Reuter, A.M., Vrinds-Geort, Y., Goldstein G., and Franchmint, P. (1986) in Immunoregulatory UCLA Symposium on Molecular and Celular Biology, eds. Goldstein, G., Wigzell, H. and Bach, J.F. (Liss, New York), inpress; Sunshine, G.H., Basch, R.S., Coffey, R.G., Cohen, K.W., Goldstein, G. and Hadden, J.W. (1978) J. Immunol. 120 1594-1599.

The complete forty-nine amino acid sequence of bovine TP (bTP) has been determined, as described in Audhya, T. Schlesinger, P.H. and Goldstein, G. (1981) Biochemistry 20 6195-6200, and the biological activity has been shown to reside in the pentapeptide ARY-LYS-ASP-VAL-TYR, named thymopentin (TP-5) and described in Goldstein G., Scheid, M.P., Boyse, E.A., Schlesinger, D.H. and Van Wauwe J. (1979) Science 204, 1309-1310 and Weksler, et al., J. Exp. Med 148-996-1006 as well as in U. S. Patent Nos. 4,190,646 and 4,261,886. Additionaly U.S. Pat. Nos. 4,361,673 4,420,424 and 4,629,723 describe various peptides asserted to have activity similar to thymopoietin. The above cited articles and patents are incorporated herein by reference.

While extensive work has been done in connection with bovine TP as well as synthetic peptides mimicking bovine TP, to date however, human thymopoietin has not been isolated from human thymus tissue and, of course, the protein has not been completely sequenced or synthesized.

## Summary of the Invention

The present invention relates to the protein human thymopoietin, substantially free of non-human thymopoietin native material i.e., free of such material associated with the protein in its natural environment in the human thymus. In particular, the present invention relates to such isolated or synthesized thymopoietin having the amino acid residue sequence:
-GLY-LEU-PRO-LYS-GLU-VAL-PRO-ALA-VAL-LEU-THR-LYS-GLY-LYS-LEU-LYS-SER-GLU-LEU-VAL-ALA-ASN-GLY-VAL-THR-LEU-PRO-ALA-GLY-GLU-MET-ARG-LYS-ASP-VAL-TYR-VAL-GLU-LEU-TYR-LEU-GLN-HIS-LEU-THR-ALA-LEU-HIS-.

Additionaly, the present invention relates to a therapeutic composition of matter comprising a therapeutically effective amount of the hTP having the above sequence and which may include among other ingredients, a pharmaceutically acceptible carrier. Specifically, such therapeutically effective amount of polypeptide may be at least about 1-1000$\mu$g/kg of body weight.

In a further aspect of this invention the therapeutic composition described above may be used in the treatment of various conditions related to the reported biological activity of hTP such as for example,

conditions resulting from relative or absolute T-cell deficiencies, from excess neuromuscular activity, or from uncontrolled proliferation of the thymus-responsive lymphocytes. The composition may be employed to induce certain desirable reactions related to the biological activity of hTP such as to induce bone marrow cells to develop the characteristics of thymus derived lymphocytes or to enhance the cellular immune responses mediated by T cells.

## Brief Description of the Drawings

Figure 1 illustrates, graphically, the result of Sephedex G-75 molecular sieve chromatography of extracts of human thymus with measurement of immunoreactive material by RIA for bTP. The lower molecular weight peaks were further purified to yield hTP;

Figure 2 illustrates graphically the result of separation of CNBr digest of hTP dissolved in 0.1% orthophosphoric acid on a $C_{18}$ reverse-phase column (Vydec 218TP54, 46 mmx25cm) at a flow rate of 2 ml/min. The peaks are designated I, II, and III. The gradient line represents the presence of organic solvent; and

Figure 3 illustrates graphically the repetitive yields of phenylthiohydantoin-derivatized amino acids during automated Edman degradation of hTP through 46 cycles; and

Figure 4 illustrates the full sequences of amino acid residues for hTP as contrasted to hSP, bTP, and bSP. The ten species specific residues shared by TP and SP within each species are shown with heavy stippling in the human sequences. Within the active site region (32-36), bTP and hTP are identified, but bSP and hSP differ at position 34.

## Detailed Description of the Invention

As used herein the following abbreviations shall apply: TP, thymopoietin; hTP, human TP; bTP, bovine TP; TP-5, thymopentin [synthetic TP-(32-36) pentapeptide]. Further, throughout this disclosure, the amino acid components of the peptides and certain materials used in their preparation are identified by abbreviations for convenience. These abbreviations are as follows:

| Amino Acid | Abbreviation |
| --- | --- |
| L-alanine | ALA |
| D-alanine | D-ALA |
| L-arginine | ARG |
| D-arginine | D-ARG |
| L-aspartic acid | ASP |
| D-aspartic acid | D-ASP |
| L-glutamic acid | GLU |
| D-glutamic acid | D-GLU |
| D-glutamine | D-GLN |
| L-histidine | HIS |
| D-histidine | D-HIS |
| L-isoleucine | ILE |
| D-isoleucine | D-ILE |
| L-leucine | LEU |
| D-leucine | D-LEU |
| L-lysine | LYS |
| D-lysine | D-LYS |
| α-methylalanine | AIB |
| L-phenylalanine | PHE |
| D-phenylalanine | D-PHE |
| L-proline | PRO |
| L-tryptophan | TRP |
| D-tryptophan | D-TRP |
| L-valine | VAL |
| D-valine | D-VAL |

In its broadest aspect this invention is concerned with human thymopoietin in a form substantially free of native material, i.e. either isolated from human thymus or synthesized and to therapeutic compositions employing the same.

Isolation and Characterization of Human Tymopoietin

The scheme for isolation of hTP from human thymus is best illustrated by the Flow Sheet, Scheme 1, below:

Scheme 1

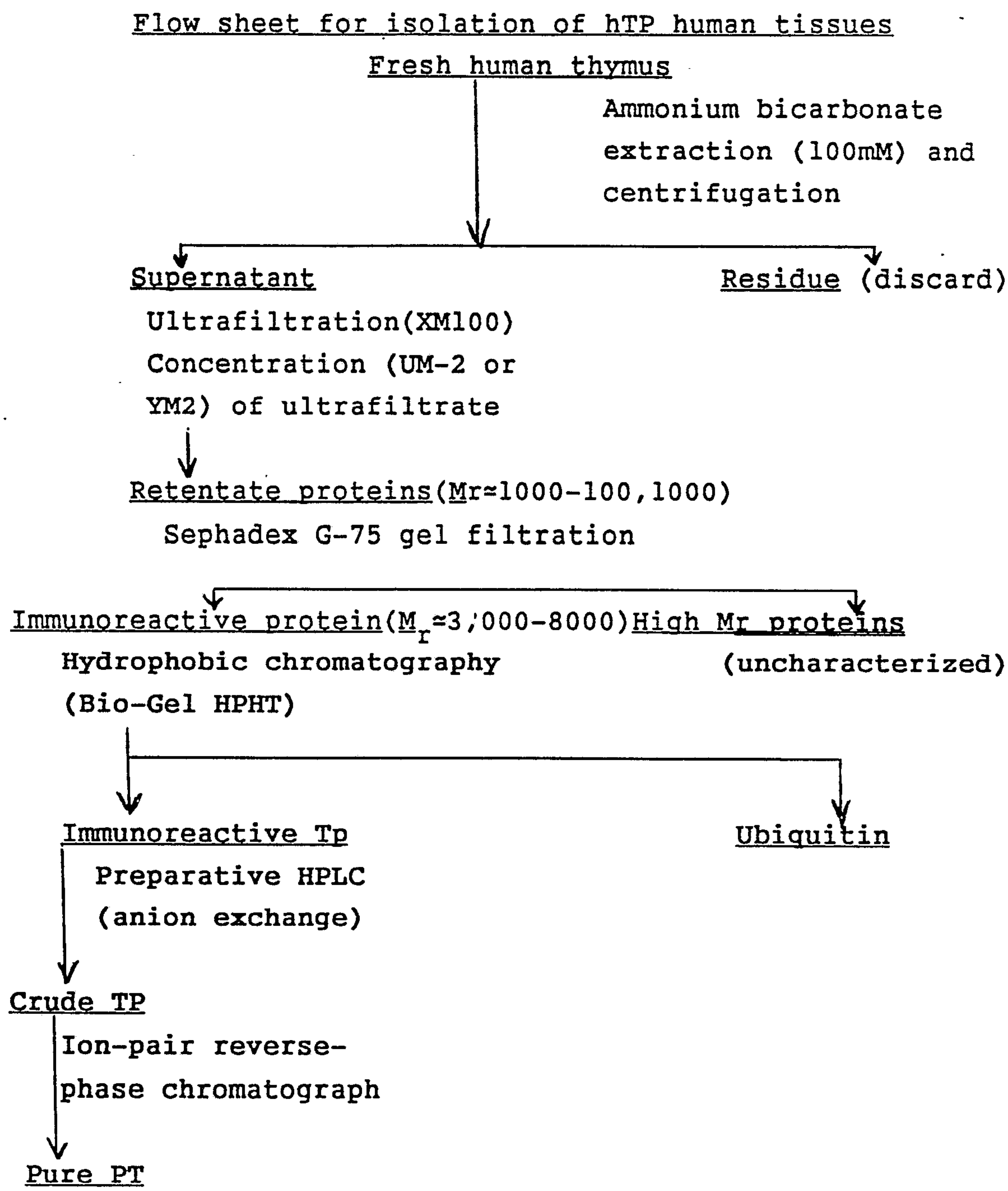


Human thymus obtained from pediatric surgical specimens and human spleen from surgical or autopsy specimens were trimmed and stored at-35°C. Separation materials and apparatus were purchased commercially: Sephadex (Pharmacia), hydroxyapatite (Bio-Gel HPHT; Bio-Rad), and preparative large-pore anion-exchange column (2.5 x 25 cm; DE 500, particle size 40-60 μ) for HPLC (Separation Industries,

Metuchen, NJ).

The purification of hTP was monitored by using a RIA for bTP that was cross-reactive with hTP (Goldstein, G. (1976) J. Immunol.117, 690-692 and Lisi, P.J. Teipel, J. W., Goldstein, G. & Schiffman, M. (1980) Clin. Chem. Acta 107, 111-119). Briefly, bTP was labeled with $^{125}I$ by the Bolton-Hunter method (Bolton, A.E. & Hunter, W.M. (1973) Biochem. J. 133, 529-539), and the radiolabeled bTP was purified as described (Audhya, T., Talle, M.A. & Goldstein, G. (1984) Arch. Biochem. Biophys. 234, 167-177). Anti-bTP antibody (diluted 1:10,000) was $^{125}I$-labeled bTP (10,000 cpm), in the presence or absence of various peptide fractions in phosphate buffer, were incubated for 8 hr. at 22° C. Separation of bound and free bTP was with 24% polyethylene glycol at room temperature, followed by centrifugation of 2000 rpm for 15 min. The bound bTP was assayed in a LKB $\gamma$ spectrometer.

A 714-g batch of human thymus was extracted in ice-cold 100 mM ammonium bicarbonate [25% (net weight) containing 50 ng of 2-mercaptoethanol, 175 $\mu$g of phenylmethylsulfonyl fluoride, and 375 $\mu$g of EDTA per ml) by homogenization in a Waring Blender (Dynamics, New Hartford, CT). After centrifugation at 14,000 x g for 10 min. at 4° C, the supernatant was filtered through cheese cloth and stored at 4° C after addition of 0.1% thimerosal and 0.1% sodium azide. The soluble extract was processed by ultrafiltration at 4° C using Amicon Diaflo membrane XM100 (molecular exclusion limit ≈ $M_r$ 100,000) and concentrated with Diaflo UM2 (molecular exclusion limit ≈ $M_r$ 1000), reducing the volume to 1/10th to 1/50th.

Scheme 1 above summarizes the isolation procedures. The retentate of the UM2 or YM2 filtration was chromatographed on bead-type Sephadex G-75 (particle size, 40-120$\mu$m) at room temperature on a 5 x 150 cm column in 50 mM ammonium bicarbonate. Immunoreactive fractions were lyophilized. Two major peaks containing immunoreactive material were found (Fig. 1). The lower molecular weight peak ($M_r$ 5000-8000) was processed further by hydroxyapatite column chromatography using Bio-Gel HPHT (2.5 x 60 cm column: 5 mM sodium phosphate buffer, pH 6.8), the sample being applied in the same buffer. Elution was with 30 ml of the same buffer and then with 35 ml of 50 mM phosphate buffer. Immunoreactive fractions were pooled, concentrated on an Amicon Diaflo UM2 membrane, desalted on an 0.6 x 30 cm Sephadex G-25 (fine) column in 10 mM ammonium bicarbonate (pH 8.0), and lyophilized.

hTP was subjected to HPLC using a 2.5 x 25 cm preparative DE 500 anion exchange column. A linear solvent gradient started with 20 mM Tris acetate buffer (pH 6.0) and finished with 80% dilution of the same buffer with 500 mM sodium acetate, pH 6.0. Immunoreactive fractions were pooled, dialyzed using Spectrapor-6 (molecular exclusion limit ≈ $M_r$ 1000) and lyophilized. Further chromatography was on a Vydac $C_{18}$ reverse-phase column (218TP54) with a linear gradient starting with 50 mM ammonium formate (pH 6.5) and finishing with 60% dilution with acetonitrile. The fractions eluted by HPLC were partly evaporated, lyophilized, and reconstituted in 500 $\mu$l of 50 mM ammonium bicarbonate (pH 8.0). Volumes of 25$\mu$l were tested in duplicate by RIA with pooling and lyophilization of immunoreactive fractions.

Yields from the purification steps are seen in Table 1.

Table 1.

| Summary of purification of hTP | | | | | |
|---|---|---|---|---|---|
| Purification Step | Total protein,mg | Immunoreactive protein | | Fold purifcat. | Rec. % |
| | | mg | % | | |
| | | hTP | | | |
| Ultrafiltered protein | 68,8000 | 309.5 | 0.45 | 1 | 100 |
| Gel Filt. of G-75 | 2,950 | 76.9 | 2.6 | 6 | 25* |
| Hydrophob. Chromotog. | 181.2 | 32.6 | 18 | 40 | 11 |
| Preparative HPLC | 3.4 | 2.8 | 82 | 182 | 0.9 |
| Rev.-phase Chromotog. | 1.4 | 1.3 | 93 | 207 | 0.42 |

*Lower molecular weight peak

Amino acid analysis was performed with a Liquimat III amino acid analyzer following acid hydrolyses in 5.7 M HCl containing 0.5% 2-mercaptoethanol for 22 hr at 100° C (Speckman, D.H., Stein, W.H. & Moore, S. (1958) Anal. Chem. 30, 1190-1206).

Maleated hTP was prepared by adding maleic anhydride in 1,4-dioxane (100 µl) at a 20-fold excess over the free amino groups of the polypeptides (500 µg), which were dissolved in 100 mM sodium borate (pH 9.3). Maleic anhydride was added stepwise over a 4-hr period, ;H 9.3 being maintained with 6 M NaOH. The maleated protein was then desalted on Bio-Gel P-2 in 100 mM $NH_4HCO_3$ (pH8.2) and lyophilized.

Tryptic digestions of maleated hTP were carried out in 0.2 M N-ethylmorpholine acetate buffer (pH 8.1) at 37°C for 6 hr. Bovine pancreatic trypsin (diphenylcarbamyl chloride-treated; 8400 Nα-benzoly-L-arginine ethyl ester units per mg of protein, Sigma type XI) was added to a final enzyme/substrate ratio of 1:100 (on a weight basis). The pH was maintained at 8.5 during the digestion and then lowered to a 2.0 by addition of 10 mM HCl to terminate the reaction. The mixture was then lyophilized.

hTP (200 pmol) were cleaved with CNBr in 150 µl of 70% formic acid for 23 hr at room temperature in the dark; a 200-fold molar excess of CNBr over the methionine content in the sample was used. The volume was then reduced to 60 µl under $N_2$ and applied directly on to the glass filter of the gas-phase sequencer (Hunkapiller, M. W., Hewick, R.M., Dryer, W. J. & Hood, L. (1983) Methods Enzymol. 91, 399-413).

The Trypsin-Digested and CNBr-Cleaved Peptides were purified in the following manner. The tryptic peptides were dissolved in 0.1% orthophosphoric acid (pH 2.2) and were centrifuged at 15,600 x g for 5 min; the supernatant was applied to the $C_{18}$ reverse-phase column [Vydac 218TP54, 46mm x 25cm, 5-µm particle size,) The Separation Group, Hesperia, CA)] for HPLC (LDC gradient module with spectromonitor II and CI-10 computing integrator attached to a LKB Redirac fraction collector 2112). A linear elution gradient was used starting with 0.1% phosphoric acid and finishing with 80% acetonitrile. High-purity-grade acetonitrile was obtained from Burdick and Jackson (Muskegon, MI) and distilled in glass. Phosphoric acid (1%) was filtered through a Millipore type HA 0.45-µm filter (Millipore). All solvents were degassed for 20 min under vacuum with stirring.

Automated sequence analyses on intact hTP and tryptic and cyanogen bromide fragments of hTP were performed by gas-phase sequencing that used a model 470A applied biosystems gas-phase sequencer with Polybrene as carrier and a standard single-coupling sing-cleavage program. The resulting phenylthiohydantoin-derivatized amino acids were identified by HPLC with a 1084B Hewlett Packard high-pressure liquid chromatograph (Schlesinger, D.H. (1983) Methods Enzymol. 91, 494-502).

C-terminal analysis of hTP (150 µg each) was performed with yeast carboxypeptidase Y (2 nmol) dissolved in 100 mM sodium acetate (pH 6.0) at 37°C. At various periods, aliquots were removed from the digest and added to tubes containing µl of glacial acetic acid, and the mixture was the lyophilized. The sample was dissolved in 0.2 M sodium citrate (pH 2.2) and applied to the amino acid analyzer. Enzyme and peptide blanks were also run.

As a result of the above methods, approximately 1.4 kg of freshly frozen human thymus yielded 1.3 mg of immunoreactive hTP (recovery = 0.4%; Table 1). This product had one major band and few minor bands on isoelectric gel focusing. The isoelectric point of the major band was 6.1 ± 0.2.

The amino acid composition is shown in Table 2.

Table 2.

Amino acid composition of hTP (molar ratios)

| Amino Acids | Found | Mean ± SK* |
|---|---|---|
| Cysteic acid | 0 | ND |
| Aspartic acid | 2 | 2.29 ± 0.12 |
| Threonine | 3 | 3.11 ± 0.23 |
| Serine | 1 | 0.81 ± 0.19 |
| Glutamic acid | 6 | 5.55 ± 0.22 |
| Proline | 3 | 2.80 ± 0.30 |
| Glycine | 3 | 2.28 ± 0.27 |
| Alanine | 4 | 4.25 ± 0.10 |
| Valine | 6 | 6.23 ± 0.30 |
| Methionine | 1 | 0.87 ± 0.13 |
| Isoleucine | 0 | 0.1 ± 0.11 |
| Leucine | 9 | 8.72 ± 0.36 |
| Tyrosine | 2 | 1.90 ± 0.12 |
| Phenylalanine | 0 | 0.16 ± 0.11 |
| Lysine | 5 | 4.92 ± 0.31 |
| Histidine | 2 | 1.76 ± 0.12 |
| Arginine | 1 | 1.07 ± 0.13 |

ND, not detected.

*Average of two determinations (24 and 76 hr). Serine was increased by 10% and threonine by 5% to compensate for destruction by acid. Hydrolysis was performed with constant-boiling HCl at 110°C *in vacuo*.

Cyanogen bromide cleavage of hTP produced three distinct fractions (Fig. 2), and the amino acid sequence results for each are summarized in Table 3.

Table 3.

| Methods of sequence determination for the various regions of hTP | |
|---|---|
| Method of Sequencing | Residues Identified |
| N-terminal degradation | |
| Native TP CNBr cleavage | 1-46 |
| Peak I | 1-11 |
| Peak II | 32-46 |
| C-terminal carboxypeptidase method | 44-48 |
| Peak III | 37-47 |
| Peak IV | No sequence obtained |
| Peak V | 18-30 |
| C-terminal carboxypeptidase method | 45-48 |

The structural Determination of hTP was carried out by. Automated N-terminal sequencing of 240 pmol of hTP through 46 cycles of the Edman degradation which gave a repetitive yield (average yield per cycle) of 93.4% and identified each of the 46 N-terminal residues (Fig. 3). The timed release of amino acids from the C terminus of intact hTP with carboxypeptidase Y (Table 4) provided an unequivocal C-terminal tetrapeptide sequence with a two-amino acid overlap (residues 45 and 46) with the N-terminal sequence. Partial sequence of hTP was confirmed by the structure determination of CNBr fragment I (Fig. 2), which encompassed residues 32-40 and which includes the active site. In addition, the amino acid composition of intact hTP (Table 2) accounted for all of the amino acids found by sequence analysis.

Table 4.

| Release of free amino acids with time from the C terminus with carboxypeptidase Y | | | | | | |
|---|---|---|---|---|---|---|
| | Amino acid yield, nmol | | | | | |
| Amino acid | 100s | 120s | 150s | 200s | 250s | 300s |
| Thr | - | - | - | 75 | 210 | 263 |
| Ala | - | - | 88 | 258 | 276 | 288 |
| Leu | - | 148 | 275 | 283 | 418 | 503 |
| His | - | 289 | 310 | 300 | 308 | 299 |

<u>Summary of Isolation of Characterization of hTP</u>

Previously, the cross-reactivity of anti-bTP antibodies was used to detect and isolate the closely related polypeptide bSP from bovine spleen (Audhya, T., Schlesinger, D.H. & Goldstein, G. (1981) Bio-chemistry 20, 6195-6200). Similarly, antibodies to bTP proved cross-reactive with hTP, and the bTP RIA was used to monitor the isolation of hTP from extracts of human thymus. The isolation involved tissue extraction in aqueous solvent, ultrafiltration for apprximate sizing, hydrophobic chromatography, and reverse-phase HPLC. Approximately 50% of the immunoreactive material in each extract was present in a higher molecular weight form that probably represents a biologically inactive precursor form (Steiner, D.F. & Oyer, P.E. (1967) Proc. Natl. Acad. Sci. USA 57, 473-480). This was separated during molecular sieving on Sephadex G-75.

Despite simplification of the isolation procedure, the recovery of hTP was very low (0.42%), the purified material representing a 207-fold purification (Table 1). Large losses of hTP occurred during HPLC. Purity of

hTP was established by polyacrylamide gel electrophoresis, HPLC, and amino acid sequence (see Fig. 3).

Residues 1-46 were determined by automated Edman degradation with a gas-phase sequencer, and residues 1-11 and 32-46 were confirmed by Edman degradation of two CNBr-cleavage fragments. This provided independent confirmation of the important active-site region, residues 32-36. The C-terminal sequence 44-48 was determined by time-release carboxypeptidase digestion.

Referring to Figure 4, hTP and human splenin (hSP, as is disclosed in a copending European patent application No. 88304579.1 were very similar in amino acid sequence, each being 48-amino acid linear polypeptides differing in only 4 residues, positions 23, 34, 43, and 47. They were also similar to bTP and bSP (bovine spleen), with 10 residues being similar between hTP and hSP but different from the common residues in bTP and bSP at these positions. These comparisons confirm that the amino acid sequences of hTP and hSP were indeed determined and indicate that gene conversion must have occurred during evolution to maintain the parallel sequence evolution of these two polypeptides within each species. Other important gene systems that utilize gene conversion include immunoglobulin(Schreier, P.H., Bothwell, A.L.M., Muller-Hile, B. & Baltimore, D. (1981) Proc. Natl. Acad. Sci. U.S.A. 78, 4495-4499) and the major histocompatibility complex (Steinmetz, M. & Hood, L. (1983) Science 222, 727-733) genes. Maintenance of parallel evolution of regions of TP and SP outside the active-site region (residues 32-36) implies that these C-and N-terminal regions of the molecule must also have an important function, with similar requirements for both TP and SP being maintained within each species.

Residues 32-36 of bTP are known to represent the active site, with the corresponding synthetic TP-(32-36) pentapeptide TP-5 having the biological activities of TP in animals and man (Goldstein, G., Scheid, M.P., Boyse, E.A., Schlesinger, D.H. & Van Wauwe, J. (1979) Science 204, 1309-1310, Audhya, T., Scheid, M.P. & Goldstein, G. (1984) Proc. Natl Acad. Sci. U.S.A. 81, 2847-2849). The present studies establish that the active site of hTP (residues 32-36) is identical with that of bTP, so that synthetic TP-5 (Arg-Lys-Asp-Val-Tyr) also represents the human active site.

It is contemplated that the peptide of this invention, in addition to being isolated from human thymus may also be produced by the intervention of the hand of man e.g., either recumbently using genetic techniques or synthetically using chemical synthesis. Conveniently, the peptides may be prepared following the solid-phase synthetic technique initially described by Merrifield in JACS, 85, 2149-2154 (1963). Such methods are also disclosed in certain of the prior art patents referred to above. Other techniques may be found, for example in M. Bodanszky, et al., Peptide Synthesis, John Wiley & Sons, second edition, 1976, as well as in other reference works known to those skilled in the art. Appropriate protective groups usable in such synthesis and their abbreviations will be found in the above texts, as well as in J.F.W. McOmie, Protective Groups in Organic Chemistry, Plenum Press, New York, 1973. Both of these books are incorporated herein by reference. The common protective groups used herein are t-butyloxycarbonyl (BOC), benzyl (BZL), t-amyloxycarbonyl (AOC), tosyl (TOS), o-bromo-phenylmethoxycarbonyl (BrZ), 2-6 dichlorobenzyl ($BZLCl_2$), and phenylmethoxycarbonyl (Z or CBZ).

Because of the immunomodulatory characteristics of the subject peptides, they are therapeutically useful in the treatment of humans and animals, since they have the capability of inducing the differentiation of lymphopoietic stem cells in the haemopoietic tissues into thymus-derived cells (T cells) which are capable of involvement in the immune response of the body. As a result, the subject peptides are considered to have multiple therapeutic uses.

Primarily, since the compounds have the capability of carrying out certain of the indicated functions of the thymus, they have application in various thymic function and immunity areas. One such application is in the treatment of DiGeorge Syndrome, a condition in which there is congenital absence of the thymus. Administration of one of the subject peptides to a sufferer from DiGeorge Syndrome will assist in overcoming this deficiency. Those of skill in the immunological art can readily determine the appropriate regimen for administration (preferably parenterally) and can determine the effective amount of one of the subject peptides for treatment of DiGeorge Syndrome.

Additionally, the subject peptides are considered useful in assisting the collective immunity of the body, in that they will increase or assist in therapeutic stimulation of cellular immunity and thereby are useful in the treatment of diseases involving chronic infection, such as fungal or mycoplasma infections, tuberculosis, leprosy, acute and chronic and viral infections and the like.

The subject compounds are generally considered to be useful in any area in which cellular immunity is an issue and particularly where there are deficiencies in immunity such as in the Di-George syndrome mentioned above. Thus, where there is an excess of antibody production due to unbalanced T cells and B cells, the subject peptides can correct this condition by stimulating T cell production. Accordingly, they are expected to be of therapeutic use in certain autoimmune diseases in which damaging antibodies are produced, such as systemic lupus erythematosis, rheumatoid arthritis, or the like.

In their broadest application, the subject compounds are useful for regulating the immune system of a subject, human or animal, in need of such regulation. As used herein, the term "regulate" means that the subject compounds cause the immune system to return from an abnormal, diseased state to a normal, balanced state. While this regulation may well find great application in the correction of immunological deficiencies (e.g., DiGeorge syndrome), it is also applicable to correct conditions of excess immunological activity (e.g., autoimmune diseases). The present invention therefore includes methods for regulating the immune system of a subject in need of such regulation which comprises administering to said subject an immunoregulatorally-effective amount of one of the subject compounds, as well as pharmaceutical compositions for practicing these methods.

The present invention provides a method for treatment of conditions resulting from relative or absolute T cell deficiencies in a subject (human or animal) having such a condition which comprises administering to the subject a therapeutically-effective amount of hTP. The invention also provides a method for treatment of conditions resulting from relative or absolute deficiencies of the thymus of a subject which comprises administering to said subject a therapeutically-effective amount of the hTP. As used herein, the term "therapeutically-effective amount" means an amount which is effective to treat conditions resulting from T cell deficiencies, or deficiencies of the thymus, respectively. The invention also provides a method for inducing lymphopoietic stem cells of a subject to develop the characteristics of thymus-derived lymphocytes which comprises administering to the subject an effective inducing amount of the hTP. The invention further provided pharmaceutical compositions for practicing those methods.

To prepare the pharmaceutical compositions of the present invention, the hTP is combined as the active ingredient in intimate admixture with a pharmaceutical carrier according to conventional pharmaceutical compounding techniques. This carrier may take a wide variety of forms depending on the form of preparation desired for administration, e.g., sublingual, rectal, nasal, oral, or parenteral. In preparing the compositions in oral dosage form, any of the usual pharmaceutical media may be employed, such as for example, water, oils, alcohols, flavoring agents, preservatives, coloring agents and the like in the case or oral liquid preparation (e.g., suspensions, elixirs, and solutions) or carriers such as starches, sugars, diluents, granulating agents, lubricants, binders, disintegrating agents, and the like in the case of oral solid preparations (e.g., powders, capsules, and tablets). Controlled release forms may also be used. Because of their ease in administration, tablets and capsules represent the most advantageous oral dosage unit form, in which case solid pharmaceutical carriers are obviously employed. If desired, tablets may be sugar coated or enteric coated by standard techniques.

For parenteral products, the carrier will usually comprise sterile water, although other ingredients to aid solubility or for preservation purposes (for example) may be included. Injectable suspensions may also be prepared, in which case appropriate liquid carriers, suspending agents, and the like may be employed.

The hTP is generally active when administered parenterally in amounts above about 1 μg/kg of body weight. For treatment of DiGeorge Syndrome, the peptides may be administered parenterally from about 0.1 to about 10 mg/kg body weight. Generally, the same range of dosage amounts may be used in treatment of the other diseases or conditions mentioned where immunodeficiency is to be treated. Larger amounts (e.g., about 10-100 mg/kg body weight) are useful for suppressing excess immune activity.

## Claims

1. Human thymopoietin, substantially free of non-human thymopoietin native matter.

2. The human thymopoietin of claim 1 having the amino acid residue sequence.
-GLY-LEU-PRO-LYS-GLU-VAL-PRO-ALA-VAL-LEU-THR-LYS-GLY-LYS-LEU-LYS-SER-GLU-LEU-VAL-ALA-ASN-GLY-VAL-THR-LEU-PRO-ALA-GLY-GLU-MET-ARG-LYS-ASP-VAL-TYR-VAL-GLU-LEU-TYR-LEU-GLN-HIS-LEU-THR-ALA-LEU-HIS-

3. The human thymopoietin of claim 1 or claim 2 extracted from human thymus.

4. The human thymopoietin of claim 1 or claim 2 chemically synthesized.

5. A therapeutic composition of matter comprising a therapeutically effective amount of the human thymopoietin of any one of claims 1 to 4.

6. The therapeutic composition of matter of claim 5 further comprising a pharmaceutically acceptable carrier.

7. The therapeutic composition of matter of claim 5 or claim 6 wherein the therapeutically effective amount of polypeptide is at least about 1 μg/kg of body weight.

8. The human thymopoietin of any one of claims 1 to 4 or the therapeutic composition of matter of any one of claims 5 to 7 for use in:
the treatment of conditions resulting from relative or absolute T-cell deficiencies;
inducing bone marrow cells to develop the characteristics of the thymus-derived lymphocytes;
affecting the immune response in the body to assist in the correction of relative or absolute deficiencies of the thymus;
the treatment of conditions resulting from excess neuromuscular activity;
inhibiting the uncontrolled proliferation of thymus-responsive lymphocytes; or
enhancing the cellular immune responses mediated by T cells.

FIG-1

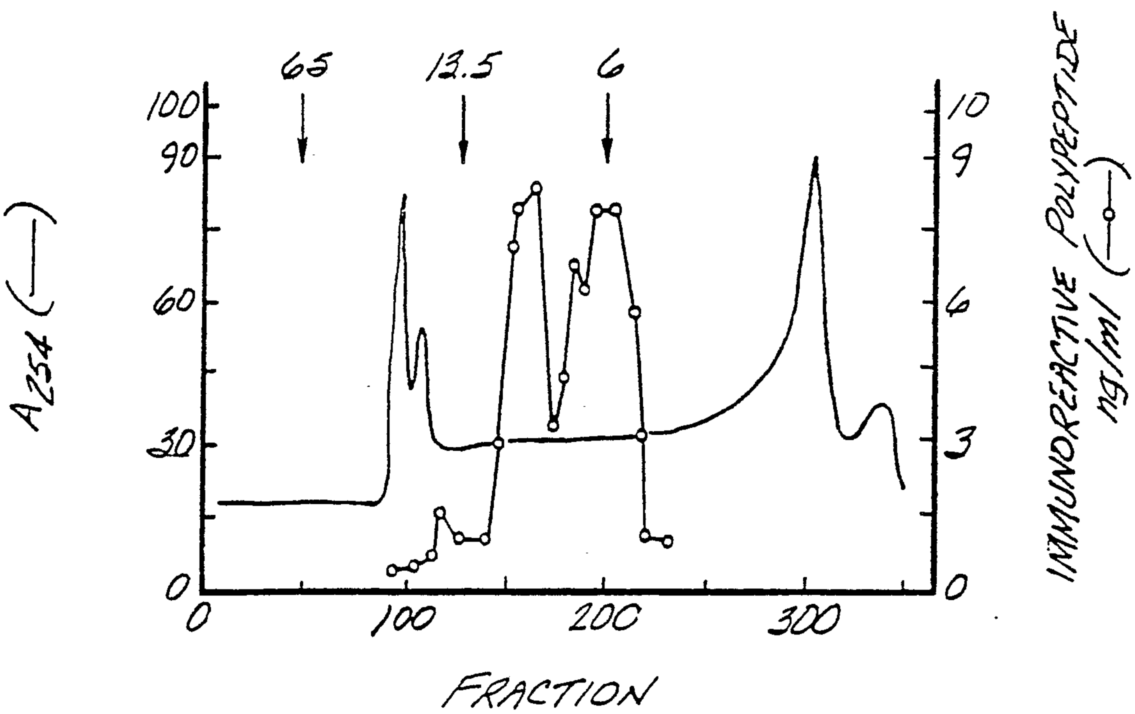
65
13.5
6
100
90
60
30
0
10
9
6
3
0
$A_{254}$ (—)
IMMUNOREACTIVE POLYPEPTIDE ng/ml (-o-)
0
100
200
300
FRACTION

FIG-2

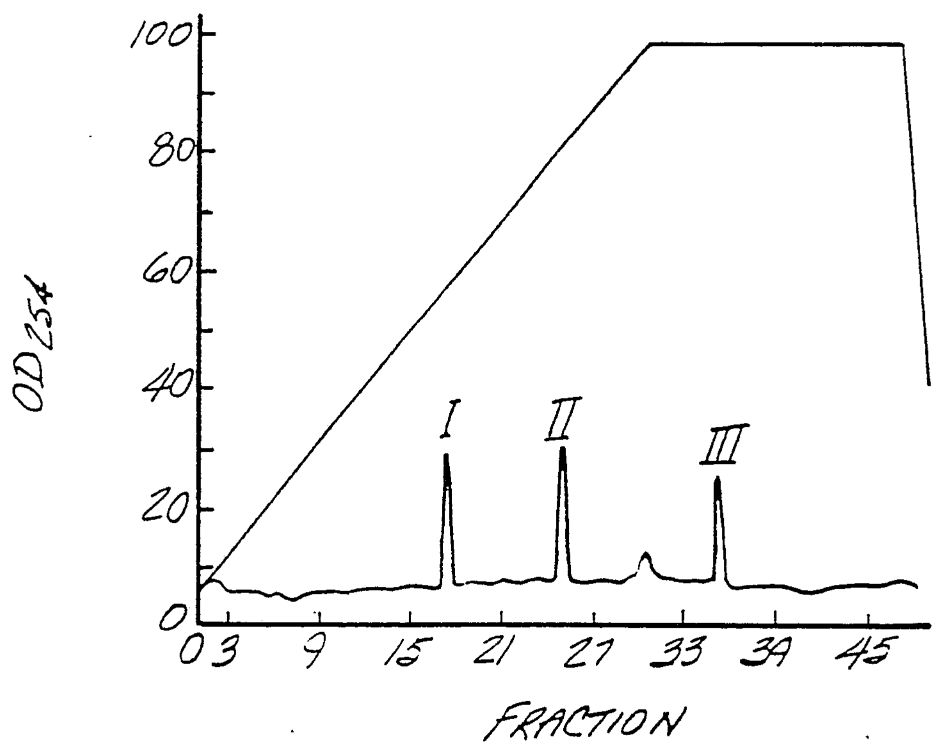
100
80
60
40
20
0
$OD_{254}$
I
II
III
0 3 9 15 21 27 33 39 45
FRACTION

FIG-3

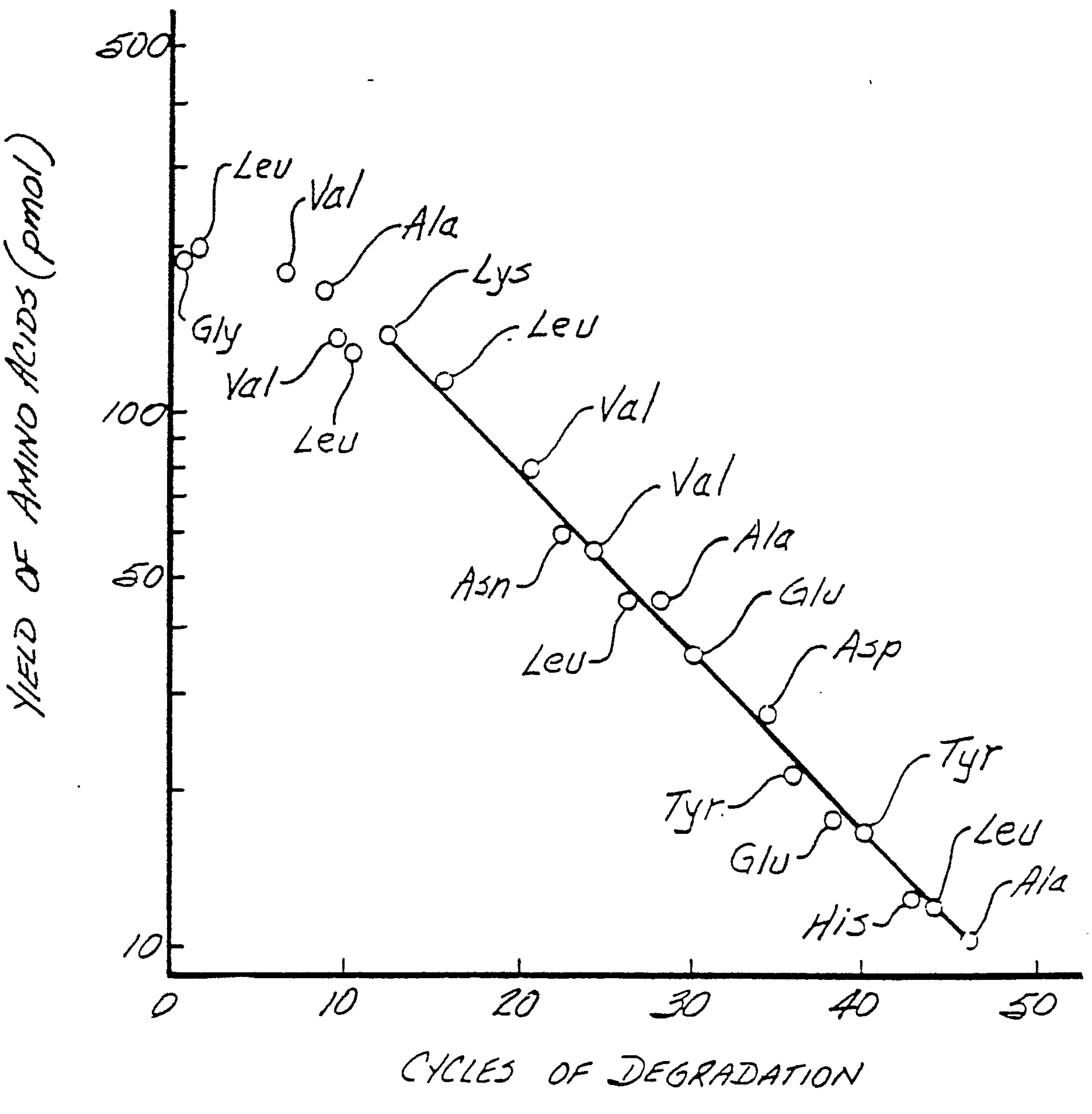

500
100
50
10
YIELD OF AMINO ACIDS (pmol)
Leu
Gly
Val
Ala
Lys
Val
Leu
Leu
Val
Val
Asn
Leu
Ala
Glu
Asp
Tyr
Tyr
Glu
His
Leu
Ala
0
10
20
30
40
50
CYCLES OF DEGRADATION

# FIG-4

| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| hSP | GLY | LEU | PRO | LYS | GLU | VAL | PRO | ALA | VAL | LEU | THR | LYS | GLN | LYS | LEU | LYS | SER | GLU | LEU | VAL | ALA | ASN | ASN | VAL | THR |
| hTP | GLY | LEU | PRO | LYS | GLU | VAL | PRO | ALA | VAL | LEU | THR | LYS | GLN | LYS | LEU | LYS | SER | GLU | LEU | VAL | ALA | ASN | GLY | VAL | THR |
| bTP | PRO | GLU | PHE | LEU | GLU | ASP | PRO | SER | VAL | LEU | THR | LYS | GLU | LYS | LEU | LYS | SER | GLU | LEU | VAL | ALA | ASN | ASN | VAL | THR |
| bSP | PRO | GLU | PHE | LEU | GLU | ASP | PRO | SER | VAL | LEU | THR | LYS | GLU | LYS | LEU | LYS | SER | GLU | LEU | VAL | ALA | ASN | ASN | VAL | THR |

| | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 |
|---|---|---|---|---|---|---|---|---|---|---|---|---|---|---|---|---|---|---|---|---|---|---|---|---|
| hSP | LEU | PRO | ALA | GLY | GLU | MET | ARG | LYS | ALA | VAL | TYR | VAL | GLU | LEU | TYR | LEU | GLN | SER | LEU | THR | ALA | GLU | HIS | |
| hTP | LEU | PRO | ALA | GLY | GLU | MET | ARG | LYS | ASP | VAL | TYR | VAL | GLU | LEU | TYR | LEU | GLN | HIS | LEU | THR | ALA | LEU | HIS | |
| bTP | LEU | PRO | ALA | GLY | GLU | GLN | ARG | LYS | ASP | VAL | TYR | VAL | GLU | LEU | TYR | LEU | GLN | SER | LEU | THR | ALA | LEU | LYS | ARG |
| bSP | LEU | PRO | ALA | GLY | GLU | GLN | ARG | LYS | GLU | VAL | TYR | VAL | GLU | LEU | TYR | LEU | GLN | HIS | LEU | THR | ALA | LEU | LYS | ARG |